# EUROPEAN PATENT SPECIFICATION

(11) **EP 1 624 871 B1**
(45) Date of publication and mention of the grant of the patent: **15.07.2009**
(21) Application number: 04785539.0
(22) Date of filing: 13.05.2004
(51) Int. Cl.: A61K 31/455, A61K 31/00, A61K 31/403, A61K 31/437, A61K 31/538, A61P 3/06

(54) **METHOD OF TREATING ATHEROSCLEROSIS, DYSLIPIDEMIAS AND RELATED CONDITIONS AND PHARMACEUTICAL COMPOSITIONS**
VERFAHREN ZUR BEHANDLUNG VON ATHEROSKLEROSE, DYSLIPIDÄMIE UND VERWANDTEN ERKRANKUNGEN UND PHARMAZEUTISCHE ZUSAMMENSETZUNGEN
METHODE DE TRAITEMENT DE L'ATHEROSCLEROSE, DYSLIPIDEMIE ET ETATS RELATIFS ET COMPOSITIONS PHARMACEUTIQUES

(30) Priority: 15.05.2003 US 470665 P
(43) Date of publication of application: 15.02.2006
(73) Proprietor: Merck & Co., Inc. (a New Jersey corp.), Rahway, N.J. 07065 (US); Merck Frosst Canada Ltd., Kirkland, Québec H9H 3L1 (CA)
(72) Inventor: CHENG, Kang, Rahway, NJ 07065-0907 (US); WATERS, M., Gerard, Rahway, NJ 07065-0907 (US); METTERS, Kathleen, M., Kirkland, Québec H9H 3L1 (CA); O'NEILL, Gary, Kirkland, Québec H9H 3L1 (CA)
(74) Representative: Horgan, James Michael Frederic
(86) International application number: PCT/US2004/014980
(87) International publication number: WO 2004/103370

(56) References cited:
- WO-A-01/79169
- WO-A-02/08186
- WO-A-96/32942
- WO-A-02/094830
- KANG C. ET AL: 'Antagonism of the prostaglandin D-2 receptor 1 suppresses nicotinic acid-induced vasodilation in mice and humans' PNAS vol. 103, no. 17, 25 April 2006, pages 6682 - 6687

## Description

### BACKGROUND OF THE INVENTION

Niacin or nicotinic acid (pyridine-3-carboxylic acid) is a drug commonly known for its effect in elevating serum levels of high density lipoproteins (HDL). However, nicotinic acid is frequently associated with cutaneous vasodilation, sometimes called flushing. This side effect is caused by the nicotinic acid-induced release of prostaglandin D2 in the skin and is so severe that many patients discontinue nicotinic acid treatment. The present invention relates to the treatment of atherosclerosis, dyslipidemias, diabetes and related conditions by administering nicotinic acid or another nicotinic acid receptor agonist in combination with a compound that reduces or eliminates the cutaneous vasodilation that otherwise occurs, such that treatment can progress without substantial flushing. This is achieved in humans by administering nicotinic acid or a nicotinic acid receptor agonist and a compound that antagonizes the DP receptor.

Different subtypes of receptors interact with prostaglandin D2. One prostaglandin D2. receptor is referred to as "DP" and another prostaglandin D2 receptor is known as "CRTH2". The present invention utilizes antagonism of the DP receptor to prevent, minimize or reduce flushing that otherwise may occur.

Consequently one object of the present invention is to eliminate or reduce substantial flushing (frequency and/or severity) as a side effect during the treatment of humans for atherosclerosis, dyslipidemia, diabetes and related conditions using nicotinic acid or another nicotinic acid receptor agonist.

Another object of the present invention is to provide combination therapy for atherosclerosis that minimizes side effects generally.

Yet another object is to provide a fixed combination pharmaceutical composition for oral use.

These and other objects will be apparent from the description provided herein.

### BRIEF DESCRIPTION OF THE DRAWINGS

The invention is illustrated in connection with the figures appended hereto in which:
Figure 1 is a graph that shows that Compound D inhibits prostaglandin D2-induced vasodilation in mice;
Figure 2 is a graph that shows that Compound D inhibits nicotinic acid induced vasodilation in mice.

Figure 3 is a graph that shows that other selected compounds inhibit nicotinic acid-induced vasodilation in mice.

### DETAILED DESCRIPTION OF THE INVENTION

Niacin or nicotinic acid (pyridine-3-carboxylic acid) is a drug commonly known for its effect in the elevation of high density lipoproteins (HDL) levels, as well as other beneficial alterations of the lipid profile (lowering very low density (VLDL), low density lipoprotein (LDL), triglycerides, free fatty acids (FFA) and lipoprotein(a) [Lp(a)]). Nicotinic acid raises HDL levels when administered to humans in therapeutically effective doses, such as about 50 mg to as high as about 8 grams per day. However, nicotinic acid is frequently associated with cutaneous vasodilation, also called flushing. Flushing typically entails a reddening of the skin, accompanied by warmth, itchiness or irritation. It can be extremely unpleasant, and can be so severe that many patients discontinue nicotinic acid treatment. The present invention relates to the treatment, prevention or reversal of atherosclerosis and the other diseases and conditions described herein, with nicotinic acid or a salt or solvate thereof, or another nicotinic acid receptor agonist without substantial flushing. This is achieved in humans by administering nicotinic acid or a salt or solvate thereof, or another nicotinic acid receptor agonist and a compound that antagonizes the DP receptor, thus preventing, reducing or minimizing the flushing effect in it frequency and/or severity.

There are at least two receptors that interact with prostaglandin D2, referred to as "DP" and "CRTH2". The present invention is primarily concerned with nicotinic acid or nicotinic acid receptor agonists used in combination with antagonists of the DP receptor.

The invention that is of interest is a pharmaceutical composition of claim 1 for use in treating atherosclerosis in a human patient in need of such treatment in amounts that are effective for treating atherosclerosis in the absence of substantial flushing.

Another aspect of the invention that is of interest relates to a pharmaceutical composition of claim 1 for use in raising serum HDL levels in a human patient in need of such treatment, said composition being effective for raising serum HDL levels in the patient in the absence of substantial flushing.

Another aspect of the invention that is of interest relates to a pharmaceutical composition of claim 1 for use in treating dyslipidemia in a human patient in need of such treatment in amounts that are effective for treating dyslipidemia in the absence of substantial flushing.

Another aspect of the invention that is of interest relates to a pharmaceutical composition of claim 1 for use in reducing serum VLDL or LDL levels in a human patient in need of such treatment in amounts that are effective for reducing serum VLDL or LDL levels in the patient in the absence of substantial flushing. '

Another aspect of the invention that is of interest relates to a pharmaceutical composition of claim 1 for use in reducing serum triglyceride levels in a human patient in need of such treatment in amounts that are effective for reducing serum triglyceride levels in the patient in the absence of substantial flushing.

Another aspect of the invention that is of interest relates to a pharmaceutical composition of claim 1 for use in reducing serum Lp(a) levels in a human patient in need of such treatment in amounts that are effective for reducing serum Lp(a) levels in the patient in the absence of substantial flushing. As used herein Lp(a) refers to lipoprotein (a).

An aspect of the invention that is of particular interest relates to each of the compositions described above wherein nicotinic acid or a salt or solvate thereof is utilized. More particularly of interest is the use of nicotinic acid. In yet a further aspect that is of interest, the DP receptor antagonist selectively modulates the DP receptor in amounts that are effective for reducing or preventing the flushing effect in the patient.

Another aspect of the invention that is of particular interest relates to each of the compositions described above wherein nicotinic acid is utilized and the DP receptor antagonist selectively modulates the DP receptor and does not substantially modulate the CRTH2 receptor.

Another aspect of the invention that is of particular interest relates to a pharmaceutical composition of claim 1 for use in treating atherosclerosis, dyslipidemias, diabetes or a related condition in a human patient in need of such treatment, said composition being administered in an amount that is effective to treat atherosclerosis, dyslipidemia, diabetes or a related condition in the absence of substantial flushing.

Compound E is particularly useful for selectively antagonizing DP receptors and suppressing the flushing effect: 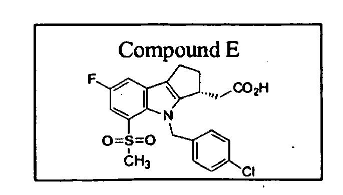 as well as the pharmaceutically acceptable salts and solvates thereof.

Atherosclerosis as used herein refers to a form of vascular disease characterized by the deposition of atheromatous plaques containing cholesterol and lipids on the innermost layer of the walls of large and medium-sized arteries. Atherosclerosis encompasses vascular diseases and conditions that are recognized and understood by physicians practicing in the relevant fields of medicine. Atherosclerotic cardiovascular disease, including restenosis following revascularization procedures, coronary heart disease (also known as coronary artery disease or ischemic heart disease), cerebrovascular disease including multi-infarct dementia, and peripheral vessel disease including erectile dysfunction, are all clinical manifestations of atherosclerosis and are therefore encompassed by the terms "atherosclerosis" and "atherosclerotic disease."

"Dyslipidemia" is used in the conventional sense to refer to abnormal levels of plasma lipids, such as HDL (low), LDL (high), VLDL (high), triglycerides (high), lipoprotein (a) (high), FFA (high) and other serum lipids, or combinations thereof. It may be an uncomplicated condition or part of a particular related disease or condition such as diabetes (diabetic dyslipidemia), metabolic syndrome and the like. Thus, uncomplicated dyslipidemias as well as those that are associated with underlying conditions are included in the present invention.

The term "patient" includes mammals, especially humans, who use the instant active agents for the prevention or treatment of a medical condition. Administering the drugs to the patient includes both self-administration and administration to the patient by another person. The patient may be in need of treatment for an existing disease or medical condition, or may desire prophylactic treatment to prevent or reduce the risk of onset of atherosclerosis.

The term "therapeutically effective amount" is intended to mean that amount of drug that will elicit the desired biological or medical response. As an example, nicotinic acid is often administered at doses from about 50 mg to about 8 grams each day.

The terms "prophylactically effective amount" and "amount that is effective to prevent" refer to that amount of drug that will prevent or reduce the risk of occurrence of the biological or medical event that is sought to be prevented. In many instances, the prophylactically effective amount is the same as the therapeutically effective amount.

The invention described herein includes the administration of the compounds and compositions described herein to prevent or reduce the risk of occurrence, or recurrence where the potential exists, of a coronary heart disease event, a cerebrovascular event, and/or intermittent claudication. Coronary heart disease events are intended to include CHD death, myocardial infarction (i.e., a heart attack), and coronary revascularization procedures. Cerebrovascular events are intended to include ischemic or hemorrhagic stroke (also known as cerebrovascular accidents) and transient ischemic attacks. Intermittent claudication is a clinical manifestation of peripheral vessel disease. The term "atherosclerotic disease event" as used herein is intended to encompass coronary heart disease events, cerebrovascular events, and intermittent claudication experienced one or more non-fatal atherosclerotic disease events are those for whom the potential for recurrence of such an event exists.. It is intended that persons who have previously.

Accordingly, the instant invention also provides a pharmaceutical composition of claim 1 for use in preventing or reducing the risk of a first or subsequent occurrence of an atherosclerotic disease event comprising the administration of a prophylactically effective amount of the compounds described herein to a patient at risk for such an event while preventing or minimizing substantial flushing. The patient may already have atherosclerotic disease at the time of administration, or may be at risk for developing it.

The pharmaceutical composition of claim 1 is further to be used in preventing or slowing new atherosclerotic lesion or plaque formation, and preventing or slowing the progression of existing lesions or plaques, as well as to causing the regression of existing lesions or plaques, while preventing or minimizing substantial flushing.

Accordingly, one aspect of this invention involves a pharmaceutical composition of claim 1 for use in halting or slowing the progression of atherosclerosis, including halting or slowing atherosclerotic plaque progression, comprising administering a therapeutically effective amount of any of the DP antagonists described herein in combination with nicotinic acid or another nicotinic acid receptor agonist to a patient in need of such treatment. This method also includes halting or slowing progression of atherosclerotic plaques existing at the time the instant treatment is begun (i.e., "existing atherosclerotic plaques"), as well as halting or slowing formation of new atherosclerotic plaques in patients with atherosclerosis.

Another aspect of this invention involves a pharmaceutical composition of claim 1 for use in preventing or reducing the risk of atherosclerotic plaque rupture comprising administering a prophylactically effective amount of any of the compounds described herein along with nicotinic acid or another nicotinic acid receptor agonist to a patient in need of such treatment. Rupture as used herein refers to the breaking loose of plaque, which can become lodged in blood vessels. A further aspect of this invention involves a pharmaceutical composition of claim 1 for use in preventing or reducing the risk of developing atherosclerosis, comprising administering a prophylactically effective amount of the compounds described herein to a patient in need of such treatment.

Another aspect of the invention relates to a pharmaceutical composition of claim 1 for use in treating or preventing atherosclerosis, dyslipidemias or a related condition comprising pretreating a human patient in need of such therapy with a flush-inhibiting or reducing effective amount of a DP receptor antagonist, thereafter treating said patient with nicotinic acid, a salt or solvate thereof, or another nicotinic acid receptor agonist in an amount that is effective to treat or prevent said atherosclerosis, dyslipidemia or related condition in the absence of substantial flushing.

Yet another aspect of the invention relates to the pharmaceutical composition of claim 1 further comprising pre-treating or treating the patient with an HMG Co-A reductase inhibitor.

Another aspect of the invention relates to a pharmaceutical composition of claim 1 for use in treating or preventing the conditions noted above wherein the HMG Co-A reductase inhibitor is simvastatin.

One aspect of the methods described herein relates to the use of nicotinic acid or another nicotinic acid receptor agonist compound in an amount that is effective for achieving the results described herein, and a DP receptor antagonist that selectively modulates the DP receptor without substantially modulating the CRTH2 receptor. Thus, the DP receptor antagonist has an affinity at the DP receptor (i.e., Kᵢ) that is at least about 10 times higher (a numerically lower Kᵢ value) than the affinity at the CRTH2 receptor. Any compound that selectively interacts with DP according to these guidelines is deemed "DP selective".

The phrase "in the absence of substantial flushing" refers to the side effect that is often seen when nicotinic acid is administered in therapeutic amounts. The flushing effect of nicotinic acid usually becomes less frequent and less severe as the patient develops tolerance to the drug at therapeutic doses, but the flushing effect still occurs to some extent. Thus, "in the absence of substantial flushing" refers to the reduced severity of flushing when it occurs, or fewer flushing events than would otherwise occur. Preferably, the incidence of flushing is reduced by at least about a third, more preferably the incidence is reduced by half, and most preferably, the flushing incidence is reduced by about two thirds or more. Likewise, the severity is preferably reduced by at least about a third, more preferably by at least half, and most preferably by at least about two thirds. Clearly a one hundred percent reduction in flushing incidence and severity is most preferable, but is not required.

The specific dosage regimen and levels for any particular patient will depend upon a variety of factors including the age, body weight, general health, sex, diet, time of administration, route of administration, rate of excretion, drug combination and the severity of the patient's condition. Consideration of these factors is well within the purview of the ordinarily skilled clinician for the purpose of determining the therapeutically effective or prophylactically effective dosage amount needed to prevent, counter, or arrest the progress of the condition. It is expected that the compounds described herein will be administered on a daily basis for a length of time appropriate to treat or prevent the medical condition relevant to the patient, including a course of therapy lasting months, years or the life of the patient.

One or more additional active agents may be administered with the compounds described herein. The additional active agent or agents can be lipid modifying compounds or agents having other pharmaceutical activities, or agents that have both lipid-modifying effects and other pharmaceutical activities. Examples of additional active agents which may be employed include but are not limited to HMG-CoA reductase inhibitors, which include statins in their lactonized or dihydroxy open acid forms and pharmaceutically acceptable salts and esters thereof, including but not limited to lovastatin (see US Patent No. 4,342,767), simvastatin (see US Patent No. 4,444,784), dihydroxy open-acid simvastatin, particularly the ammonium or calcium salts thereof, pravastatin, particularly the sodium salt thereof (see US Patent No. 4,346,227), fluvastatin particularly the sodium salt thereof (see US Patent No. 5,354,772), atorvastatin, particularly the calcium salt thereof (see US Patent No. 5,273,995), pitavastatin also referred to as NK-104 (see PCT international publication number WO 97/23200) and rosuvastatin, also known as ZD-4522, (CRESTOR^{®}; see US Patent No. 5,260,440); HMG-CoA synthase inhibitors; squalene epoxidase inhibitors; squalene synthetase inhibitors (also known as squalene synthase inhibitors), acyl-coenzyme A: cholesterol acyltransferase (ACAT) inhibitors including selective inhibitors of ACAT-1 or ACAT-2 as well as dual inhibitors of ACAT-1 and -2; microsomal triglyceride transfer protein (MTP) inhibitors; endothelial lipase inhibitors; bile acid sequestrants; LDL receptor inducers; platelet aggregation inhibitors, for example glycoprotein IIb/IIIa fibrinogen receptor antagonists and aspirin; human peroxisome proliferator activated receptor gamma (PPARγ) agonists including the compounds commonly referred to as glitazones for example pioglitazone and rosiglitazone and, including those compounds included within the structural class known as thiazolidine diones as well as those PPARγ agonists outside the thiazolidine dione structural class; PPARα agonists such as clofibrate, fenofibrate including micronized fenofibrate, and gemfibrozil; PPAR dual α/γ agonists; vitamin B₆ (also known as pyridoxine) and the pharmaceutically acceptable salts thereof such as the HCl salt; vitamin B₁₂ (also known as cyanocobalamin); folic acid or a pharmaceutically acceptable salt or ester thereof such as the sodium salt and the methylglucamine salt; anti-oxidant vitamins such as vitamin C and E and beta carotene; beta-blockers; angiotensin II antagonists such as losartan; angiotensin converting enzyme inhibitors such as enalapril and captopril; renin inhibitors, calcium channel blockers such as nifedipine and diltiazem; endothelin antagonists; agents that enhance ABCA1 gene expression; cholesteryl ester transfer protein (CETP) inhibiting compounds, 5-lipoxygenase activating protein (FLAP) inhibiting compounds, 5-lipoxygenase (5-LO) inhibiting compounds, famesoid X receptor (FXR) ligands including both antagonists and agonists; Liver X Receptor (LXR)-alpha ligands, LXR-beta ligands, bisphosphonate compounds such as alendronate sodium; cyclooxygenase-2 inhibitors such as rofecoxib and celecoxib; and compounds that attenuate vascular inflammation.

Cholesterol absorption inhibitors can also be used in the present invention. Such compounds block the movement of cholesterol from the intestinal lumen into enterocytes of the small intestinal wall, thus reducing serum cholesterol levels. Examples of cholesterol absorption inhibitors are described in U.S. Patent Nos. 5,846,966, 5,631,365, 5,767,115, 6,133,001, 5,886,171, 5,856,473, 5,756,470, 5,739,321, 5,919,672, and in PCT application Nos. WO 00/63703, WO 00/60107, WO 00/38725, WO 00/34240, WO 00/20623, WO 97/45406, WO 97/16424, WO 97/16455, and WO 95/08532. The most notable cholesterol absorption inhibitor is ezetimibe, also known as 1-(4-fluorophenyl)-3(R)-[3(S)-(4-fluorophenyl)-3-hydroxypropyl)]-4(S)-(4-hydroxyphenyl)-2-azetidinone, described in U.S. Patent Nos. 5,767,115 and 5,846,966.

Therapeutically effective amounts of cholesterol absorption inhibitors include dosages of from about 0.01 mg/kg to about 30 mg/kg of body weight per day, preferably about 0.1 mg/kg to about 15 mg/kg.

For diabetic patients, the compounds used in the present invention can be administered with conventional diabetic medications. For example, a diabetic patient receiving treatment as described herein may also be taking insulin or an oral antidiabetic medication. One example of an oral antidiabetic medication useful herein is metformin.

### Dosage Information

Nicotinic acid as used herein refers to pyridine-3-carboxylic acid. However, salts and solvates of nicotinic acid are also included for use in the present invention, and numerous pharmaceutically acceptable salts and solvates of nicotinic acid are useful in the present invention. Alkali metal salts, in particular, sodium and potassium, form salts that are useful as described herein. Likewise alkaline earth metals, in particular, calcium and magnesium, form salts that are useful as described herein. Various salts of amines, such as ammonium and substituted ammonium compounds also form salts that are useful as described herein. Similarly, solvated forms of nicotinic acid are useful within the present invention. Examples include the hemihydrate, mono-, di-, tri- and sesquihydrate. Of particular interest for use in the present invention is the free acid, pyridine-3-carboxylic acid.

DP antagonists, as described herein, are useful for reducing or preventing the flushing effect in mammalian patients, particularly humans, at dosages ranging from as low as about 0.01 mg/kg/day to as high as about 100 mg/kg/day, administered in single or divided daily doses. Preferably the dosages are from about 0.1 mg/day to as high as about 1.0 g/day, in single or divided daily doses.

The dose of nicotinic acid that is useful as described herein ranges from as low as about 50 mg/day to as high as about 8 g/day, in single or divided daily doses. Lower dosages can be used initially, and dosages increased to further minimize the flushing effect.

The dosages of nicotinic acid receptor agonists other than nicotinic acid vary within wide limits. Generally, nicotinic acid receptor agonists that are useful for treating atherosclerosis will be administered in amounts ranging from as low as about 0.01 mg/kg/day to as high as about 100 mg/kg/day, in single or divided doses. A representative dosage is about 0.1 mg/day to about 2 g/day.

The compounds used in the present invention can be administered via any conventional route of administration. The preferred route of administration is oral.

The nicotinic acid, salt or solvate thereof, or other nicotinic acid receptor agonist and the DP antagonist can be administered together or sequentially in single or multiple daily doses, e.g., bid, tid or qid, without departing from the invention. If particularly long sustained release is desired, such as a sustained release product showing a release profile that extends beyond 24 hours, dosages may be administered every other day. However, single daily doses are preferred. Likewise, morning or evening dosages can be utilized.

### Pharmaceutical Compositions

The pharmaceutical compositions described herein are generally comprised of nicotinic acid or another nicotinic acid receptor agonist, a DP receptor antagonist and a pharmaceutically acceptable carrier.

Examples of suitable oral compositions include tablets, capsules, troches, lozenges, suspensions, dispersible powders or granules, emulsions, syrups and elixirs. Examples of carrier ingredients include diluents, binders, disintegrants, lubricants, sweeteners, flavors, colorants, preservatives, and the like. Examples of diluents include, for example, calcium carbonate, sodium carbonate, lactose, calcium phosphate and sodium phosphate. Examples of granulating and disintegrants include corn starch and alginic acid. Examples of binding agents include starch, gelatin and acacia. Examples of lubricants include magnesium stearate, calcium stearate, stearic acid and talc. The tablets may be uncoated or coated by known techniques. Such coatings may delay disintegration and thus, absorption in the gastrointestinal tract and thereby provide a sustained action over a longer period.

In one embodiment of the invention, nicotinic acid, a salt or solvate thereof, or another nicotinic acid receptor agonist is combined with the DP receptor antagonist and the carrier to form a fixed combination product. This fixed combination product may be a tablet or capsule for oral use.

More particularly, in another embodiment of the invention, nicotinic acid, or a salt or solvate thereof, or another nicotinic acid receptor agonist (about 1 to about 1000 mg) and the DP antagonist (about 1 to about 500 mg) are combined with the pharmaceutically acceptable carrier, providing a tablet or capsule for oral use.

Sustained release over a longer period of time may be particularly important in the formulation of nicotinic acid pharmaceutical compositions. Sustained release tablets are particularly preferred. For example, a time delay material such as glyceryl monostearate or glyceryl distearate may be employed. The dosage form may also be coated by the techniques described in the U.S. Patent Nos. 4,256,108; 4,166,452 and 4,265,874 to form osmotic therapeutic tablets for controlled release.

Other controlled release technologies are also available and are included herein. Typical ingredients that are useful to slow the release of nicotinic acid in sustained release tablets include various cellulosic compounds, such as methylcellulose, ethylcellulose, propylcellulose, hydroxypropylcellulose, hydroxyethylcellulose, hydroxypropylmethylcellulose, microcrystalline cellulose, starch and the like. Various natural and synthetic materials are also of use in sustained release formulations. Examples include alginic acid and various alginates, polyvinyl pyrrolidone, tragacanth, locust bean gum, guar gum, gelatin, various long chain alcohols, such as cetyl alcohol and beeswax.

A sustained release tablet that is of particular interest utilizes nicotinic acid in combination with one or more of the cellulosic compounds noted above, compressed into a sustained release tablet to form a polymer matrix. The DP antagonist compound can be incorporated into the blend before compression, or can be coated onto the outer surface of the matrix.

In an embodiment that is of more interest, the nicotinic acid and matrix-forming material are combined and compressed to form a sustained release core, and the DP antagonist compound is blended with one or more coating agents and coated onto the outer surface of the core.

Optionally and of even more interest is a tablet as described above, further coated with an HMG Co-A reductase inhibitor, for example, simvastatin. This particular embodiment thus contains three active ingredients, the HMG Co-A reductase inhibitor and the DP antagonist, which may be releasable substantially upon ingestion, and the nicotinic acid which may be releasable over a longer period of time as described above.

Typical release time frames for sustained release tablets in accordance with the present invention range from about 1 to as long as about 48 hours, preferably about 4 to about 24 hours, and more preferably about 8 to about 16 hours.

Hard gelatin capsules constitute another solid dosage form for oral use. Such capsules similarly include the active ingredients mixed with carrier materials as described above. Soft gelatin capsules include the active ingredients mixed with water-miscible solvents such as propylene glycol, PEG and ethanol, or an oil such as peanut oil, liquid paraffin or olive oil.

Aqueous suspensions are also contemplated as containing the active material in admixture with excipients suitable for the manufacture of aqueous suspensions. Such excipients include suspending agents, for example sodium carboxymethylcellulose, methylcellulose, hydroxypropylmethylcellulose, sodium alginate, polyvinylpyrrolidone, tragacanth and acacia; dispersing or wetting agents,e.g., lecithin; preservatives, e.g., ethyl, or n-propyl para-hydroxybenzoate, colorants, flavors, sweeteners and the like.

Dispersible powders and granules suitable for preparation of an aqueous suspension by the addition of water provide the active ingredients in admixture with a dispersing or wetting agent, suspending agent and one or more preservatives. Suitable dispersing or wetting agents and suspending agents are exemplified by those already mentioned above.

Syrups and elixirs may also be formulated.

Another pharmaceutical composition that is of particular interest is a sustained release tablet that is comprised of nicotinic acid or a salt or solvate thereof, compound E and an HMG Co-A reductase inhibitor in combination with a pharmaceutically acceptable carrier.

Yet another pharmaceutical composition that is of more particular interest is a sustained release tablet that is comprised of nicotinic acid, compound E and simvastatin in combination with a pharmaceutically acceptable carrier.

Yet another pharmaceutical composition that is of even more interest is comprised of nicotinic acid, compound E and simvastatin in combination with a pharmaceutically acceptable carrier.

The term "composition", in addition to encompassing the pharmaceutical compositions described above, also encompasses any product which results, directly or indirectly, from the combination, complexation or aggregation of any two or more of the ingredients, active or excipient, or from dissociation of one or more of the ingredients, or from other types of reactions or interactions of one or more of the ingredients. Accordingly, the pharmaceutical composition of the present invention encompasses any composition made by admixing or otherwise combining the compounds, any additional active ingredient(s), and the pharmaceutically acceptable excipients.

Another aspect of the invention relates to the use of nicotinic acid or a salt or solvate thereof, or another nicotinic acid receptor agonist and a DP antagonist in the manufacture of a medicament. This medicament has the uses described herein.

More particularly, another aspect of the invention relates to the use of nicotinic acid or a salt or solvate thereof, or another nicotinic acid receptor agonist, a DP antagonist and an HMG Co-A reductase inhibitor, such as simvastatin, in the manufacture of a medicament. This medicament has the uses described herein.

In addition to nicotinic acid, which is the benchmark nicotinic acid receptor agonist, numerous nicotinic acid receptor agonists have been described. The following publications disclose compounds that are nicotinic acid receptor agonists:
Lorenzen, A. et al. Molecular Pharmacology 59: 349-357 (2001),
Lorenzen, A. et al. Biochemical Pharmacology 64: 645-648 (2002),
Soga, T. et al. Biochemical and Biophysical Research Comm. 303: 364-369 (2003),
Tunaru, S. et al. Nature Medicine 9: 352-355 (2003),
Wise, A. et al. Journal of Biological Chemistry 278: 9869-9874 (2003), and
Van Herk, T. et al Journal of Medicinal Chemistry 46: 3945-3951 (2003).

It is noted that partial agonists for the Nicotinic acid receptor, such as those disclosed in van Herk, et al. are included in the present compositions and methods of treatment.

Moreover, the nicotinic acid receptor has been identified and characterized in WO02/084298A2 published on October 24, 2002 and in Soga, T. et al., Tunaru, S. et al. and Wise, A. et al. (citations above).

### EXAMPLE 1

### (-)-[(4-Chlorobenzyl)-7-fluoro-5-methanesulfonyl)-1,2,3,4-tetrahydrocyclogenta[b]indol-3-yl]acetic acid (Compound E)

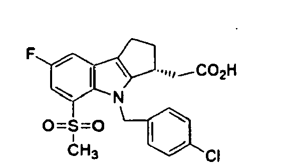

### Step 1: (+/-)-(7-Fluoro-1,2,3,4-tetrahydrocyclopenta[b]indol-3-yl)acetic acid ethyl ester.

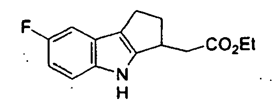

A solution of 10.00 g of 4-fluoro-2-iodoaniline, 6.57 g of ethyl 2-(2-oxocyclopentyl)acetate and 121 mg of p-toluenesulfonic acid in 100 ml of benzene was refluxed with a Dean-Stark trap under a N₂ atmosphere for 24h. After this time, the benzene was removed under distillation. Then, 60ml of DMF was added and the solution was degassed before 19 ml of Hunig's base followed by 405 mg of Pd(OAc)₂ were added successively. The solution was heated to 115°C for 3h, then cooled to room temperature. To quench the reaction, 300 ml of 1 N HCl and 200 ml of ethyl acetate were added and the mixture was filtered through Celite. The phases were separated and the acidic phase was extracted twice with 200 ml of ethyl acetate. The organic layers were combined, washed with brine, dried over anhydrous Na₂SO₄, filtered through Celite and concentrated. The crude material was further purified by flash chromatography eluting with 100% toluene, to provide the title compound.
¹H NMR (acetone-d₆) δ 9.76 (br s, 1H), 7.34 (dd, 1H), 7.03 (d, 1H), 6.78 (td, 1H), 4.14 (q, 2H), 3.57 (m, 1H), 2.85-2.55 (m, 5H), 2.15 (m, 1H), 1.22 (t, 3H).

### Step 2: (+/-)-(7-Fluoro-1,2,3,4-tetrahydrocyclopenta[b]indol-3-yl)acetic acid

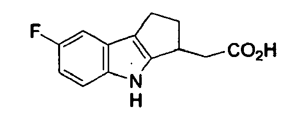

To a solution of 1.24 g of the ester from Step 1 in 14 mL of tetrahydrofuran (THF) at room temperature, 7 mL of MeOH followed by 7 mL of 2N NaOH were added. After 2.5 h, the reaction mixture was poured into a separatory funnel containing ethyl acetate (EtOAc)/1N HCl. The phases were separated and the acidic phase was extracted twice with EtOAc. The organic layers were combined, washed with brine, dried over anhydrous Na₂SO₄ and evaporated to dryness to yield a crude oil that was used as such in the next step (>90% purity).
¹H NMR (acetone-d₆) δ 10.90 (br s, 1H), 9.77 (br s, 1H), 7.34 (dd, 1H), 7.04 (dd, 1H), 6.79 (td, 1H), 3.56 (in, 1H), 2.90-2.50 (m, 5H), 2.16 (m, 1H). MS (-APCI) m/z 232.2 (M-H)⁻.

### Step 3: (+/-)-(5-bromo-7-fluoro-1,2,3,4-tetrahydrocyclopenta[b]indol-3-yl)acetic acid

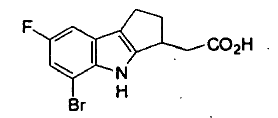

To a solution of 2.20 g of the acid from Step 2 (>90% purity) in 30 mL of pyridine, 6.85 g of pyridinium tribromide (90% purity) was added at -40°C. The suspension was stirred for 10 min at 0°C and warmed to room temperature for 30 min. Then, the solvent was removed without heating under high vacuum. The crude material was dissolved in 40 mL of AcOH and 2.88 g of Zn dust was added portion wise to the cold solution at 0°C. The suspension was stirred for 15 min at 15°C and warmed to room temperature for an additional 15 min. At this time, the reaction mixture was quenched by the addition of 1N HCl and this mixture was poured into a separatory funnel containing brine/EtOAc. The layers were separated and the organic layer was washed with water, brine, dried over anhydrous Na₂SO₄ and concentrated. This material was used without further purification in the next step.
¹H NMR (acetone-d₆) δ 10.77 (br s, 1H), 9.84 (br s, 1H), 7.09 (m, 2H), 3.60 (m, 1H), 2.95-2.65 (m, 4H), 2.56 (dd, 1 H), 2.19 (m, 1H).

### Step 4: (+/-)-[5-bromo-4-(4-chlorobenzyl)-7-fluoro-1,2,3,4-tetrahydrocyclopenta[b]indol-3-yl]-acetic acid

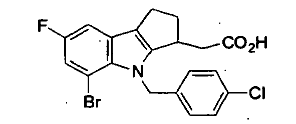

To a solution of 2.13 g of the acid from Step 3 in 10 mL of THF, a solution of diazomethane in ether was added in excess until complete consumption of the acid as monitored on TLC. Then, the solvents were removed under vacuum. To a solution of the crude methyl ester thus formed in 20 mL of DMF, 539 mg of a NaH suspension (60% in oil) was added at -78°C. The suspension was stirred for 10 min at 0°C, cooled again to -78°C and treated with 1.70 g of 4-chlorobenzyl bromide: After 5 min, the temperature was warmed to 0°C and the mixture was stirred for 20 min. At this time, the reaction was quenched by the addition of 2 mL of AcOH and this mixture was poured into a separatory funnel containing 1N HCl/EtOAc. The layers were separated and the organic layer was washed with brine, dried over anhydrous Na₂SO₄ and concentrated. The alkylated material was hydrolyzed using the procedure described in Step 2. The crude material was further purified by trituration with EtOAc/hexanes to provide the title compound.
¹H NMR (acetone-d₆) δ 10.70 (br s, 1H), 7.31 (d, 2H), 7.18 (d, 1H), 7.06 (d, 1H), 6.92 (d, 2H), 5.90 (d, 1H), 5.74 (d, 1H), 3.61 (m, 1H), 3.00-2.70 (m, 3H), 2.65 (dd, 1H), 2.39 (dd, 1H), 2.26 (m, 1H). MS (-APCI) m/z 436.3, 434.5 (M-H)⁻.

### Step 5: (+)-[5-bromo-4-(4-chlorobenzyl)-7-fluoro-1,2,3,4-tetrahydrocyclopenta[b]indol-3-yl}acetic acid

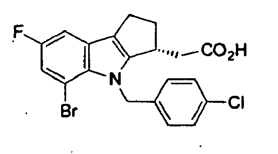

To a solution of 2.35 g of the acid of Step 4 in 130 mL of EtOH at 80°C, was added 780 µL of (S)-(-)-1-(1-naphthyl)ethylamine. The solution was cooled to room temperature and stirred overnight. The salt recovered (1.7 g) was recrystallized again with 200 mL of EtOH. After filtration, the white solid salt obtained was neutralized with 1N HCl and the product was extracted with EtOAc. The organic layer was washed with brine, dried over anhydrous Na₂SO₄ and concentrated. The material was filtered over a pad of SiO₂ by eluting with EtOAc to produce the title enantiomer. Retention times of the two enantiomers were respectively 7.5 min and 9.4 min [ChiralPak AD column, hexane/2-propanol/acetic acid (95:5:0.1)]. The more polar enantiomer was in 98% ee.
ee = 98%; Retention time = 9.4 min [ChiralPak AD column: 250 x 4.6 mm, hexanes/2-propanol/acetic acid (75:25:0.1)]; [α]_{D}²¹ = +39.2° (*c* 1.0, MeOH).

### Step 6: (-)-[4-(4-chlorobenzyl)-7-fluoro-5-(methanesulfonyl)-1,2,3,4-tetrahydrocyclopenta[b]-indol-3-yl} acetic acid and sodium salt

The acid from Step 5 (15.4 g) was first esterified with diazomethane. The sulfonylation was accomplished by mixing the ester thus formed with 16.3 g of methanesulfinic acid sodium salt and 30.2 g of CuI (I) in N-methylpyrrolidinone. The suspension was degassed under a flow of N₂, heated to 150°C and stirred for 3h, then cooled to room temperature. To quench the reaction, 500 ml of ethyl acetate and 500 ml of hexanes were added and the mixture was filtered through a pad of SiO₂ by eluting with EtOAc. The organic phases were concentrated. The crude oil was dissolved with EtOAc, washed three times with water one time with brine, dried over anhydrous Na₂SO₄, filtered and concentrated. The crude material was further purified by flash chromatography eluting with a gradient from 100% toluene to 50% toluene in EtOAc, to provide 14 g of the sulfonated ester, which was hydrolyzed using the procedure described in Step 2. The title compound was obtained after two successive recrystallizations: isopropyl acetate / heptane followed by CH₂Cl₂ / hexanes.
¹H NMR (500 MHz acetone-d₆) δ 10.73 (br s, 1H), 7.57 (d, 2H, *J*=8.8 Hz), 7.31 (m, 1H), 7.29 (m, 1H), 6.84 (d, 2H, *J*=8.8 Hz), 6.29 (d, 1H, *J_{AB}* =17.8 Hz), 5.79 (d, 1H, *J_{AB}*=17.8 Hz), 3.43 (m, 1H), 2.98 (s, 3H), 2.94 (m, 1H), 2.85-2.65 (m, 3H), 2.42 (dd, 1H, *J₁*=16.1 Hz, *J₂*=10.3 Hz), 2.27 (m, 1H). ¹³C NMR (125 MHz acetone-d₆) δ 173.0, 156.5 (d, *J*_{CF}=237 Hz), 153.9, 139.2, 133.7, 133.3, 130.0 (d, *J*_{CF}=8.9 Hz), 129.6, 128.2, 127.5 (d, *J*_{CF}=7.6 Hz), 122.2 (d, *J*_{CF}=4.2 Hz), 112.3 (d, *J*_{CF}=29.4 Hz), 111.0 (d, *J*_{CF}=22.6 Hz), 50.8, 44.7, 38.6, 36.6, 36.5, 23.3. MS (-APCI) m/z 436.1, 434.1 (M-H)⁻. ee = 97%; Retention time = 15.3 min [ChiralCel OD column: 250 x 4.6 mm, hexanes/2-propanol/ethanol/acetic acid (90:5:5:0.2)]; [α]_{D}²¹ = -29.3° (*c* 1.0, MeOH). Mp 175.0°C.

The sodium salt was prepared by the treatment of 6.45 g (14.80 mmol) of the above acid compound in EtOH (100 mL) with 14.80 mL of an aqueous 1N NaOH solution. The organic solvent was removed under vacuum and the crude solid was dissolved in 1.2L of isopropyl alcohol under reflux. The final volume was reduced to 500 mL by distillation of the solvent. The sodium salt crystallized by cooling to rt. The crystalline sodium salt was suspended in H₂O, frozen with a dry ice bath and lyophilized under high vacuum to give the title compound as the sodium salt.
¹H NMR (500 MHz DMSO-d₆) δ 7.63 (dd, 1H, *J*₁=8.5 Hz, *J*₂=2.6 Hz), 7.47 (dd, 1H, *J*₁=9.7 Hz, *J*₂=2.6 Hz), 7.33 (d, 2H, *J*=8.4 Hz), 6.70 (d, 2H, *J*=8.4 Hz), 6.06 (d, 1H, *J_{AB}*=17.9 Hz), 5.76 (d, 1H, *J_{AB}*=17.9 Hz), 3.29 (m, 1H), 3.08 (s, 3H), 2.80 (m, 1H), 2.69 (m, 1H), 2.55 (m, 1H), 2.18 (m, 2H), 1.93 (dd, 1H, *J₁*=14.4 Hz, *J₂*=9.7 Hz).

### EXAMPLE 1A

### Alternative procedure for (+/-)-[5-bromo-4-(4-chlorobenzyl)-7-fluoro-1,2,3,4-tetrahydrocyclopenta[b]indol-3-yl]acetic acid (Example 1, Step 4)

### Step 1: (+/-)-7-fluoro-1,2,3,4-tetrahydrocyclopenta[b]indol-3-yl)acetic acid dicyclohexylamine (DCHA) salt

A 0.526 M solution of 2-bromo-4-fluoroaniline in xylene along with ethyl (2-oxocyclopentyl) acetate (1.5 eq) and sulfuric acid (0.02 eq) was heated to reflux for 20 hours. Water was azeotropically removed with a Dean-Stark apparatus. The reaction was followed by NMR and after 20 hours, an 80-85% conversion to the desired imine intermediate was generally observed. The reaction mixture was washed with 1M sodium bicarbonate (0.2 volumes) for 15 minutes and the organic fraction was evaporated. The remaining syrup was distilled under vacuum (0.5 mm Hg). Residual xylenes distilled at 30°C, then excess ketone and unreacted aniline were recovered in the 50-110°C range; the imine was recovered in the 110-180°C fraction as a light brown clear liquid with 83% purity.

The imine intermediate was then added to a degased mixture of potassium acetate (3 eq), tetra-n-butylammonium chloride monohydrate (1 eq), palladium acetate (0.03 eq) and N,N-dimethylacetamide (final concentration of imine = 0.365 M). The reaction mixture was heated to 115°C for 5 hours and allowed to cool to room temperature. 3N KOH (3 eq) was then added and the mixture was stirred at room temperature for 1 hour. The reaction mixture was diluted with water (1.0 volume), washed with toluene (3x0.75 volume). The aqueous phase was acidified to pH 1 with 3N HCl and extracted with tertbutyl methyl ether (2x0.75 volume). The combined organic fractions were washed with water (0.75 volume). To the clear light brown solution was added dicyclohexylamine (1 eq) and the solution was stirred at room temperature for 16 hours. The salt was filtered, washed with ethyl acetate, tertbutyl methyl ether and allowed to dry to give the title compound. Assay: 94 A%.
1H NMR (500 mHz, CDCl3) : δ 9.24 (s, 1H), 7.16-7.08 (m, 2H), 6.82 (t, 1H), 6.2 (br, 2H), 3.6-3.5 (m, 1H), 3.04-2.97 (m, 2H), 2.88-2.70 (m, 3H), 2.66 (dd, 1H), 2.45-2.37 (m, 1H), 2.13-2.05 (m, 2.05), 1.83 (d, 4H), 1.67 (d, 2H), 1.55-1.43 (m, 4H), 1.33-1.11 (m, 6H).

### Step 2: (+/-)-(5-bromo-7-fluoro-1,2,3,4-tetrahydrocyclopenta[b]indol-3-yl)acetic acid

A slurry of the DCHA salt from Step 1 above in dichloromethane (0.241 M solution) was cooled to -20 to -15 °C. Pyridine (2 eq.) was added in one shot and to the slurry was added dropwise bromine (2.5 eq.) over 30 to 45 minutes maintaining the temperature between -20 °C and -15°C. (At about 1/3 addition of bromine, the reaction mixture was thick and an efficient stirring was needed. Eventually, at about 1/2 addition of bromine, the mixture became "loose" again.) After completion of the addition, the reaction mixture was aged for one additional hour at -15°C. Acetic acid (3.04 eq.) was then added over 5 minutes and zinc dust (3.04 eq.) was added portion wise. (A portion of zinc was added at -15 °C and the mixture was aged for about 5 minutes to ensure that the exotherm was going (about - 15°C to -10°C)). This operation was repeated with about 5 shots of zinc over about 30 min. When no more exotherm was observed, the remaining zinc was added faster. The whole operation took around 30 to 45 minutes.

After completion of the addition, the batch was warmed to room temperature, aged 1 hour and concentrated. The reaction mixture was switched to methyl t-butyl ether (MTBE, 0.8 volume) and a 10% aqueous acetic acid solution (0.8 volume) was added. The mixture (crystallization of salts, e.g pyridium) was aged at room temperature for 1 hour and filtered through solka-floc. The pad of solka-floc was rinsed with MTBE (ca. 0.2 volume) and the filtrate (biphasic, MTBE/aqueous) was transferred into an extractor. The organic phase was washed with water (0.8 volume). The MTBE extract was concentrated and switched to isopropyl alcohol (IPA, 0.25 volume) to crystallize the compound. Water (0.25 volumes) was added and the batch was aged for 1 hour. Additional water (0.33 volumes) was added over 1 hour. After completion of the water addition, the batch was aged for one additional hour, filtered, and rinse with 30/70 IPA/Water (0.15 volumes). Crystallized bromoacid was dried in the oven at +45 °C.

### Step 3: (+/-)- [5-bromo-4-(4-chlorobenzyl)-7-fluoro-1,2,3,4-tetrahydrocyclopenta[b]indol-3-yl]-acetic acid

The bromoacid of Step 2 was dissolved in dimethylacetamide (0.416 M solution) and cesium carbonate (2.5 eq.) was added in one portion. To the slurry was added in one portion 4-chlorobenzyl chloride (2.5 eq.) and the batch was heated to 50°C for 20 h. The batch was cooled to r.t. and sodium hydroxide 5N (4.00 eq.) was added over 5 minutes (temperature rose to +40 °C). The reaction was aged at 50 °C for ca. 3 hours, cooled to room temperature and transferred into an L extractor. The solution was diluted with isopropylacetate (IPAc, 2 volumes) and cooled to +15 °C. The solution was acidified with 5N HCl to pH~2. Layers were separated and the organic layer was washed with water (2x2 volumes). IPAc solution was concentrated and switched to IPA (0.8 volumes) to crystallize the product. Water (8 L) was added over 2 hours and the batch was filtered to give the title compound. The batch can be dried in the oven at +40 °C for 24 hours.

### Biology

The compounds used in the present invention that function as selective DP antagonists typically demonstrate an affinity (Kᵢ) for DP that is at least about 10 times higher (a numerically lower Kᵢ value) than the affinity (Kᵢ) for CRTH2 receptors. Typical DP antagonists used in the present invention are at least about 10-fold selective for the DP receptor over the CRTH2 receptor. More particularly, the selective DP receptor antagonist is at least about 100 fold selective for the DP receptor relative to the CRTH2 receptor. Even more particularly, the DP selective antagonist compound is at least about 800-1000 fold selective for the DP receptor over the CRTH2 receptor, i..e., the affinity (K¡) for the DP receptor is 800-1000 times higher than the affinity (K_{¡}) for the CRTH2 receptor.

As used herein when a compound "selectively modulates the DP receptor", the compound binds to and antagonizes the DP receptor at a concentration that is achievable at therapeutic doses, while not substantially modulating the CRTH2 receptor at such therapeutically achievable concentrations.

Generally the DP antagonists used herein have an affinity (Kᵢ) for the CRTH2 receptor of about 0.5 micromolar or higher. Compounds having a binding affinity for CRTH2 of about 0.5 micromolar or higher, and a selectivity for the DP receptor over CRTH2 of at least about 10 fold, are useful to inhibit the flushing effect seen when nicotinic acid is administered without such selective DP antagonists.

### Determination of the Affinity and Selectivity of Compounds at Recombinant Human DP and CRTH2 Receptors

The receptor affinity and selectivity of compounds at DP and CRTH2 was determined using radioligand binding assays as described in Abramovitz M, et al. Biochem. Biophys. Acta (2000)1483: 285-293, and Sawyer N, et al. Br. J. Pharmacol. (2002); 137: 1163-1172. Briefly, stable cell lines that individually express human DP and CRTH2 receptors were established using human embryonic kidney (HEK) 293EBNA (Epstein Barr virus Nuclear Antigen) cells (designated HEK293E cell lines). Membrane fractions prepared from these recombinant cell lines were employed in equilibrium competition radioligand binding assays to determine the affinity and selectivity of compounds at the DP and CRTH2 receptors.

DP and CRTH2 cDNAs corresponding to full length coding sequences were subcloned into the appropriate sites of the mammalian expression vector pCEP4 (Invitrogen) and expressed in HEK293E cells. Membranes were prepared by differential centrifugation (1000 x g for 10 min, then 160,000 x g for 30 min, all at 4°C) following lysis of the cells by nitrogen cavitation at 800 psi for 30 min on ice in the presence of protease inhibitors (2 mM AEBSF, 10 µM E-64, 100 µM leupeptin and 0.05 mg/mL pepstatin). The 160,000 x g pellets were resuspended in 10 mM HEPES/KOH (pH 7.4) containing 1 mM EDTA at approximately 5 to 10 mg/mL protein by Dounce homogenisation (Dounce A; 10 strokes), frozen in liquid nitrogen and stored at -80°C. Receptor binding assays were performed in a final incubation volume of 0.2 mL in 10 mM HEPES/KOH (pH 7.4), containing 1 mM EDTA, 10 mM MnCl₂ and 0.7 nM [³H]PGD₂ (200 Ci/mmol). The reaction was initiated by addition of membrane protein (approximately 30 µg for DP and 10 µg for CRTH2) from the 160,000 x g fraction. Ligands were added in dimethylsulfoxide (DMSO) which was kept constant at 1% (v/v) in all incubations. Non-specific binding was determined in the presence of 10 µM of non-radioactive PGD₂. Incubations were conducted on a mini-orbital shaker at room temperature for 60 min. The binding assay was terminated by rapid filtration through a 96-well Unifilter GF/C (Canberra Packard) prewetted in assay incubation buffer without EDTA (at 4°C) using a Tomtec Mach III 96-well semi-automated cell harvester. The filters were washed with 3 to 4 mL of the same buffer, dried for 90 min at 55°C and the residual radioactivity bound to the individual filters determined by scintillation counting with addition of 50 µL of Ultima Gold F (Canberra Packard) using a 1450 MicroBeta (Wallac) counter.

Maximum specific binding was defined as the total binding minus the non-specific binding in the absence of competitor. Specific binding was determined at each concentration of compound and was expressed as a percentage of the maximum specific binding. Sigmoidal equilibrium competition curves were constructed by expressing percentage maximum specific binding as a function of test compound concentration and analyzed by a custom designed software package employing a simplex driven non-linear least-squares curve fitting routine based on a four parameter equation to determine the inflection point (InPt). The binding affinity of the test compound was determined by calculating the equilibrium inhibition constant (Kᵢ) from the equation Kᵢ = InPt/1+([radioligand]/K_{d}), where K_{d} is the equilibrium dissociation constant for the radioligand-receptor interaction. When InPt could not be determined the IC₅₀ was used (i.e. the concentration of test compound required to inhibit 50 % of the maximum specific binding).

Generally the compounds used in the present invention demonstrate a K_{¡} for the DP receptor of from about as low as about 0.4 nM to as high as about 16.3 nM. Likewise, the compound used in the present invention generally demonstrate a K_{¡} for the CRTH2 receptor of as low as about 180 nM to as high as about 22,000 nM or even higher.

### Effect of Compounds on Nicotinic acid-Induced Vasodilation in Mice

The potency of the selective DP antagonists described herein can be demonstrated using a murine model of human nicotinic acid-induced flushing, measuring the flushing inhibitory effect. Blood flow in the mouse ear (a measure of vasodilation, a prominent component of flushing in humans) is measured after administration of nicotinic acid to mice that had been pretreated with vehicle (as a control) or a DP antagonist. Specifically, male C57BL/6 mice (~25 g) were used in the study. Five mice were evaluated in each test group. Nembutal was diluted with water to a final concentration of 5 mg/ml and injected 0.3 ml/mouse intraperitoneally. DP antagonists were dissolved in 5% hydroxypropyl β-cyclodextrin at a final concentration of 5 mg/ml and the compounds were administered intraperitoneally at a volume of 0.2 ml/mouse (~40 mpk). Nicotinic acid was dissolved in 5% hydroxypropyl β-cyclodextrin at a final concentration of 12.5 mg/ml. The nicotinic acid stock solution was adjusted to pH 7.4 with 2 N NaOH and injected 0.2 ml/mouse subcutaneously (~100 mpk).

Perfusion of mouse ear skin was monitored with a laser Doppler perfusion imager (PeriScan PIM II, Perimed, Sweden) every 30 seconds for 15 minutes starting 5 minutes prior to nicotinic acid administration. Percent changes in mean perfusion over the 10 minute period after vehicle or nicotinic acid administration were calculated and a graph of percent change in mean perfusion vs. time was generated for each animal. The area under the curve (AUC) of mean perfusion (% Δ x min) was then calculated from each graph and the results are expressed in mean AUC ± SEM for each group.

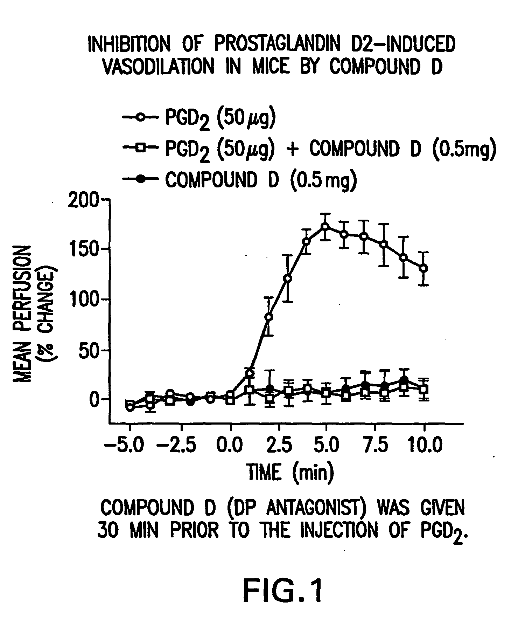

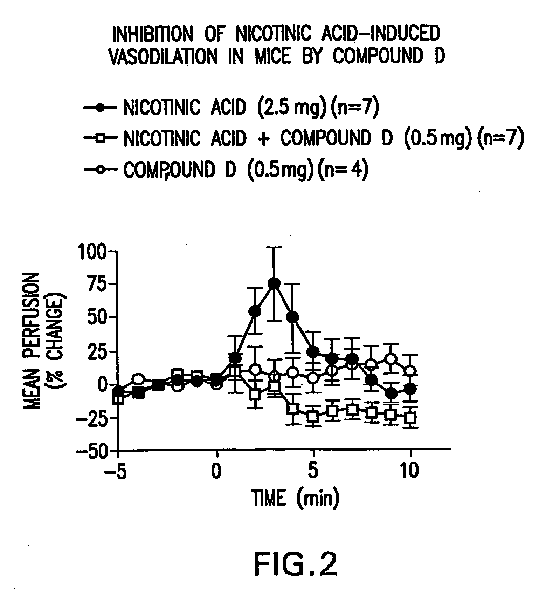

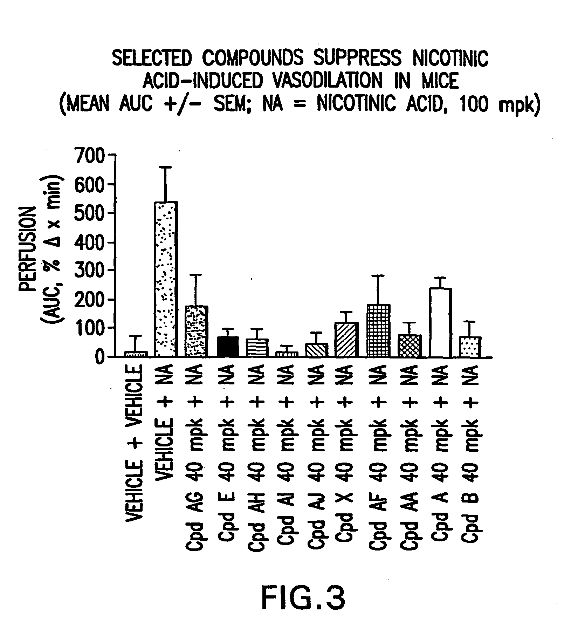

Compound D suppressed PGD-2 induced vasodilation in the mouse (Fig. 1). The DP antagonists tested suppressed nicotinic acid-induced vasodilation in the mouse; data for selected compounds is provided in Figures 2 and 3.

## Claims

1. A pharmaceutical composition comprised of compound E, or a pharmaceutically acceptable salt or solvate thereof: 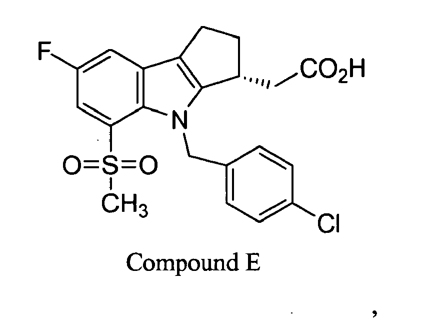 nicotinic acid or a pharmaceutically acceptable salt or solvate thereof and an HMG Co-A reductase inhibitor in combination with a pharmaceutically acceptable carrier.

2. A pharmaceutical composition in accordance with claim 1 wherein the HMG Co-A reductase inhibitor is selected from lovastatin, simvastatin, dihydroxy open-acid simvastatin, pravastatin, fluvastatin, atorvastatin, pitavastatin and rosuvastatin.

3. A pharmaceutical composition in accordance with Claim 1 wherein the HMG Co-A reductase inhibitor is simvastatin.

4. A pharmaceutical composition in accordance with any previous claim for use in therapy.

5. A pharmaceutical composition in accordance with any one of claims 1 to 3 for use in treating atherosclerosis, raising serum HDL levels, treating dyslipidemia, reducing serum VLDL or LDL levels, reducing serum triglyceride levels or reducing serum lipoprotein(a) levels.

6. A pharmaceutical composition in accordance with any one of claims 1 to 3 for use in treating atherosclerosis or dyslipidemia.

## Patentansprüche

1. Eine pharmazeutische Zusammensetzung, die Verbindung E oder ein pharmazeutisch annehmbares Salz oder Solvat davon: 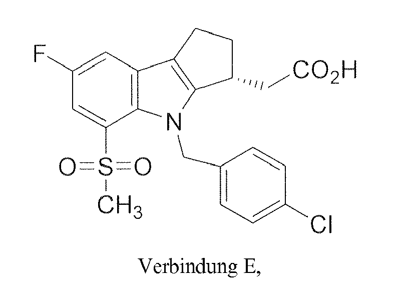 Nikotinsäure oder ein pharmazeutisch annehmbares Salz oder Solvat davon und einen HMG-Co-A-Reduktase-Inhibitor in Kombination mit einem pharmazeutisch annehmbaren Träger enthält.

2. Eine pharmazeutische Zusammensetzung gemäß Anspruch 1, wobei der HMG-Co-A-Reduktase-Inhibitor ausgewählt ist aus Lovastatin, Simvastatin, Simvastatin in der offenen Dihydroxysäureform, Pravastatin, Fluvastatin, Atorvastatin, Pitavastatin und Rosuvastatin.

3. Eine pharmazeutische Zusammensetzung gemäß Anspruch 1, wobei der HMG-Co-A-Reduktase-Inhibitor Simvastatin ist.

4. Eine pharmazeutische Zusammensetzung gemäß einem vorherigen Anspruch zur Verwendung in der Therapie.

5. Eine pharmazeutische Zusammensetzung gemäß einem der Ansprüche 1 bis 3 zur Verwendung bei der Behandlung von Atherosklerose, zur Erhöhung von Serum-HDL-Spiegeln, zur Behandlung von Dyslipidämie, zur Senkung von Serum-VLDL- oder -LDL-Spiegeln, zur Senkung von Serumtriglyceridspiegeln oder zur Senkung von Serumlipoprotein(a)-Spiegeln.

6. Eine pharmazeutische Zusammensetzung gemäß einem der Ansprüche 1 bis 3 zur Verwendung bei der Behandlung von Atherosklerose oder Dyslipidämie.

## Revendications

1. Composition pharmaceutique constituée du composé E, ou un sel ou solvate pharmaceutiquement acceptable de celui-ci : 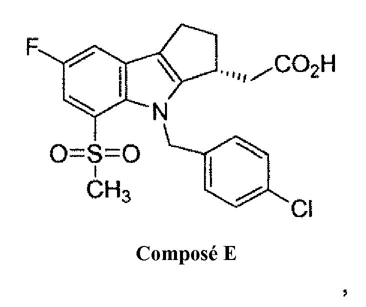 acide nicotinique ou sel ou solvate pharmaceutiquement acceptable de celui-ci et un inhibiteur de la HMG Co-A réductase en combinaison avec un vecteur pharmaceutiquement acceptable.

2. Composition pharmaceutique selon la revendication 1, dans lequel l'inhibiteur de la HMG Co-A réductase est sélectionné à partir de lovastatine, simvastatine, simvastatine d'acide ouvert en dihydroxy, pravastatine, fluvastatine, atorvastatine, pitavastatine et rosuvastatine.

3. Composition pharmaceutique selon la revendication 1, dans lequel l'inhibiteur de la HMG Co-A réductase est la simvastatine.

4. Composition pharmaceutique selon l'une quelconque des revendications précédentes en vue d'une utilisation thérapeutique.

5. Composition pharmaceutique selon l'une quelconque des revendications 1 à 3 en vue d'une utilisation dans le traitement de l'athérosclérose, l'augmentation des taux de sérums HDL, le traitement de la dyslipidémie, la réduction des taux de sérum VLDL ou LDL, la réduction des taux de sérum triglycéride ou la réduction des taux de sérum lipoprotéine(a).

6. Composition pharmaceutique selon l'une quelconque des revendications 1 à 3 en vue d'une utilisation dans le traitement de l'athérosclérose ou de la dyslipidémie.
